# EUROPEAN PATENT APPLICATION

(11) **EP 4 821 506 A1**
(43) Date of publication of application: **14.10.2026**
(21) Application number: 23960441.6
(22) Date of filing: 05.12.2023
(51) Int. Cl.: A61K 39/395, C07K 16/28, C07K 16/24

(54) **USE OF ANTI-IL-17RB ANTIBODY OR ANTIGEN-BINDING FRAGMENT THEREOF OR COMPOSITION**

(71) Applicant: Sinomab Bioscience Limited, New Territories, Hong Kong Sar PRC (HK)
(72) Inventor: HUI, Chin Wai, Pak Shek Kok, New Territories, Hong Kong (CN); LAM, Lik Hang, Pak Shek Kok, New Territories, Hong Kong (CN); LI, Weimin, Pak Shek Kok, New Territories, Hong Kong (CN); WU, Wai Chung, Pak Shek Kok, New Territories, Hong Kong (CN); CHOW, Kai Cheung, Pak Shek Kok, New Territories, Hong Kong (CN); LEUNG, Shui On, Pak Shek Kok, New Territories, Hong Kong (CN)
(74) Representative: Lorenz Seidler Gossel Part. mbB
(86) International application number: PCT/CN2023/136312
(87) International publication number: WO 2025/118126

(57) **Abstract**

Disclosed in the present application are an isolated anti-IL-17RB antibody or antigen-binding fragment thereof, or a pharmaceutical composition containing same, which are used for treating FLG-associated diseases. The anti-IL-17RB antibody or antigen-binding fragment thereof or the pharmaceutical composition containing same can reverse Th2-driven downregulation of filaggrin expression, and can thus effectively treat FLG-associated diseases.

## Description

### TECHNICAL FIELD

The present application pertains to the field of immunopharmaceuticals, and specifically relates to use of an anti-IL-17RB antibody or antigen-binding fragment thereof, or a pharmaceutical composition comprising the same, in the preparation of a medicament for treating FLG-related diseases.

### BACKGROUND

The skin covers the entire human body and protects humans from various external stimuli. Barrier impairment leads to enhanced penetration of external antigens, which easily induce skin inflammation. Persistent skin inflammation, in turn, further weakens the skin barrier function. This results in a "vicious cycle" between the skin barrier and skin immunity. Filaggrin (FLG) and its metabolites are key components maintaining skin barrier function. For example, deficiency of FLG is a major pathogenic factor in a variety of skin diseases, such as atopic dermatitis.

Atopic dermatitis (AD) is a chronic inflammatory skin disease that usually occurs in childhood and may persist into adulthood. Over the past few decades, the prevalence and incidence of atopic dermatitis have continued to increase. Research data show that the prevalence is approximately 15% to 30% in children and up to 10% in adults, making atopic dermatitis the skin disease with the highest disease burden. The pathogenesis of atopic dermatitis involves genetic susceptibility, immune and epidermal barrier dysfunction, and environmental factors. Pruritus is its primary symptom, and skin lesions can manifest from mild erythema to lichenification with severe erythroderma.

Traditional treatments for atopic dermatitis include topical administration of glucocorticoids, phototherapy, JAK inhibitors (e.g., ruxolitinib, upadacitinib), calcineurin inhibitors, PDE4 inhibitors, etc. Among others, glucocorticoids, as the main treatment modality, can provide good relief effects, but are not suitable for long-term use due to various side effects. Moreover, atopic dermatitis often relapses after discontinuation, so it is necessary to be combined withother treatment modalities to treat atopic dermatitis. The launch of upadacitinib sustained-release tablets has provided more treatment options for patients with atopic dermatitis. After two weeks of administration, the patient's skin lesions are improved, and after 16 weeks of administration, more patients achieved high-level lesion clearance.

Dupilumab (Dupixent^{™}), a fully human anti-IL-4/IL-13 monoclonal antibody jointly developed by Sanofi and Regeneron, was first approved by the FDA in March 2017 and first approved for marketing in China in June 2020. It specifically binds to the IL-4Rα subunit, thereby inhibiting the signal transduction of IL-4 and IL-13 and blocking the inflammatory responses mediated by IL-4 and IL-13, thus being useful for the treatment of atopic dermatitis. However, the results of the Phase III clinical trial of upadacitinib (NCT03738397) reported that compared with dupilumab, upadacitinib showed efficacy advantages in primary and all secondary endpoints, and upadacitinib had a faster onset of action.

In addition, the U.S. FDA approved tezepelumab (Tezspire^{™}), an anti-TSLP antibody jointly developed by AstraZeneca and Amgen, as a drug for the treatment of severe asthma in 2021. A Phase 2a randomized, double-blinded clinical study (NCT02525094) using tezepelumab in adult patients with moderate to severe atopic dermatitis was initiated in August 2015. Although the proportion of patients achieving EASI50 (50 Percent (%) Reduction From Baseline in Eczema Area and Severity Index) was higher in the tezepelumab plus TCS (topical corticosteroids) group than in the placebo plus TCS group (64.7% vs. 48.2%), the treatment difference was not statistically significant (Eric L Simpson et al., JAAD, 2019 Apr, 80(4): 1013-1021).

There is still a need for more options for the treatment of diseases associated with filaggrin downregulation.

### SUMMARY OF THE INVENTION

The inventors of the present application found in research that anti-IL-17RB antibodies can restore FLG expression in the skin of patients with allergic diseases or conditions (e.g., dermatitis), thereby improving skin barrier function and thus achieving the treatment of related diseases and conditions.

The present application relates to use of an isolated anti-IL-17RB antibody or antigen-binding fragment thereof, or a pharmaceutical composition comprising the same in the preparation of a medicament for treating FLG-related diseases, wherein the antibody comprises: HCDR1 set forth in SEQ ID NO:1, HCDR2 set forth in SEQ ID NO:2, and HCDR3 set forth in SEQ ID NO:3; and/or LCDR1 set forth in SEQ ID NO:4, LCDR2 set forth in SEQ ID NO:5, and LCDR3 set forth in SEQ ID NO:6.

Alternatively, the present application relates to an isolated anti-IL-17RB antibody or antigen-binding fragment thereof, or a pharmaceutical composition comprising the same for use in treating FLG-related diseases, wherein the antibody comprises: HCDR1 set forth in SEQ ID NO:1, HCDR2 set forth in SEQ ID NO:2, and HCDR3 set forth in SEQ ID NO:3; and/or LCDR1 set forth in SEQ ID NO:4, LCDR2 set forth in SEQ ID NO:5, and LCDR3 set forth in SEQ ID NO:6.

Alternatively, the present application relates to a method of treating FLG-related diseases, comprising administering to a subject in need thereof an isolated anti-IL-17RB antibody or antigen-binding fragment thereof, or a pharmaceutical composition comprising the same, wherein the antibody comprises: HCDR1 set forth in SEQ ID NO:1, HCDR2 set forth in SEQ ID NO:2, and HCDR3 set forth in SEQ ID NO:3; and/or LCDR1 set forth in SEQ ID NO:4, LCDR2 set forth in SEQ ID NO:5, and LCDR3 set forth in SEQ ID NO:6.

Alternatively, the present application relates to a medicament for treating FLG-related diseases, comprising an isolated anti-IL-17RB antibody or antigen-binding fragment thereof, or a pharmaceutical composition comprising the same, wherein the antibody comprises: HCDR1 set forth in SEQ ID NO:1, HCDR2 set forth in SEQ ID NO:2, and HCDR3 set forth in SEQ ID NO:3; and/or LCDR1 set forth in SEQ ID NO:4, LCDR2 set forth in SEQ ID NO:5, and LCDR3 set forth in SEQ ID NO:6.

The anti-IL-17RB antibody according to the present application exhibits good affinity for IL-17RB (interleukin-17 receptor B), is capable of reversing Th2-driven downregulation of filaggrin expression, reducing ILC2 populations in submental lymph nodes, attenuating secretion of IL-4, IL-5, and IL-13 by lymph node cells, significantly alleviating epidermal hyperplasia, inhibiting infiltration of mast cells and eosinophils, and thereby restoring skin barrier function and achieving treatment of FLG-related diseases.

In addition, the inventors also found through preclinical *in vivo* animal experiments that the anti-IL-17RB antibody according to the present application has superior efficacy in the treatment of atopic dermatitis compared to upadacitinib, especially being more effective and faster in reducing pruritus and improving skin pathology.

### BRIEF DESCRIPTION OF DRAWINGS

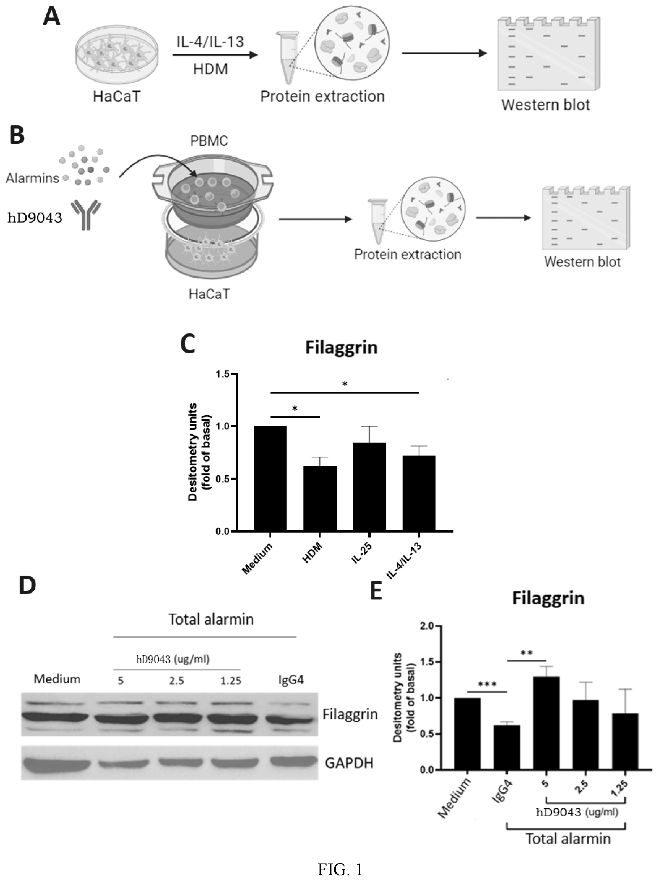

FIG. 1: Filaggrin expression in terminally differentiated HaCaT cells after treatment with cytokines and anti-IL-17RB antibody hD9043. A) Schematic diagram of direct treatment of mature HaCaT cells with different reagents; B) Schematic diagram of co-culture between PBMCs and mature HaCaT cells; C) Quantification of band intensities for filaggrin expression after direct treatment on mature HaCaT cells; D) Representative Western blots of filaggrin expression after co-culture; E) Quantification showing that 5 µg/mL anti-IL-17RB antibody hD9043 could significantly restore filaggrin levels in the presence of total alarmins. *** p<0.001, ** p<0.01, * p<0.05, as analyzed by Student's t-test. N=3.

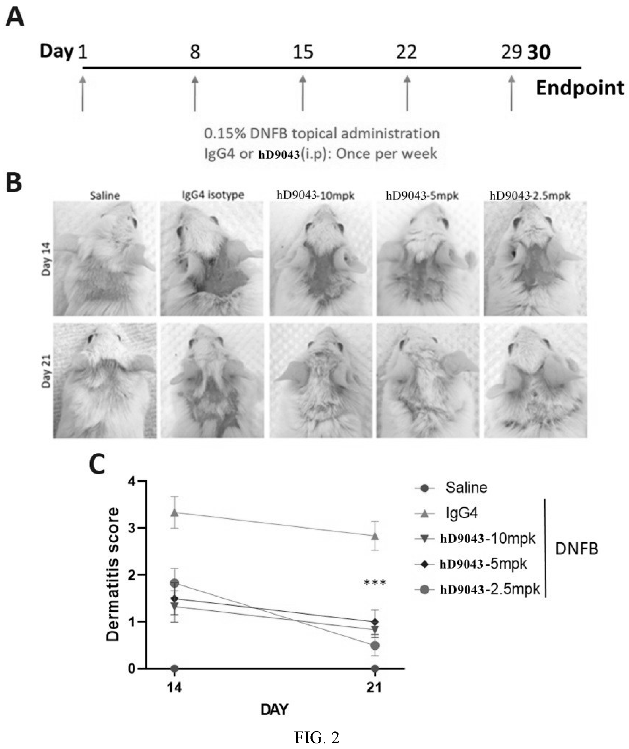

FIG. 2: Development of an atopic dermatitis animal model using DNFB. A) Treatment paradigm of topical application of DNFB and antibody treatment within one month; B) Representative images of dorsal skin of mice across different groups; C) Quantification of dermatitis scores showing that anti-IL-17RB antibody hD9043 could significantly restore skin conditions in DNFB-treated mice on Day 14 and Day 21. *** p<0.001, as analyzed by two-way ANOVA with post hoc-test. N=9.

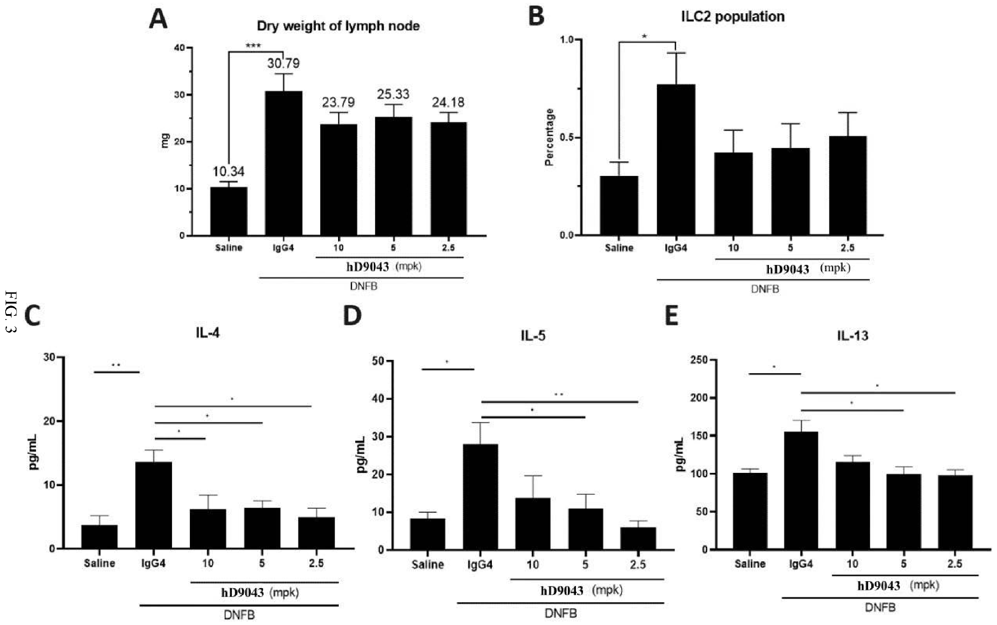

FIG. 3: Anti-IL-17RB antibody hD9043 significantly reduced Th2 immunity in submental lymph nodes. A) Dry weight of four submental lymph nodes from mice in different groups; B) ILC2 population in single isolated cells from lymph nodes; C-E) Levels of IL-4, IL-5 and IL-13 in supernatants after culturing lymph nodes for 1 day in the presence of DNFB restimulation. For A and B, *** p < 0.001, * p < 0.05 compared with the normal saline control group, as analyzed by Student's t-test. N=7-9. For C-E, ** p < 0.01, * p < 0.05 compared with the IgG4+DNFB group, as analyzed by one-way ANOVA with post-hoc test. N=6.

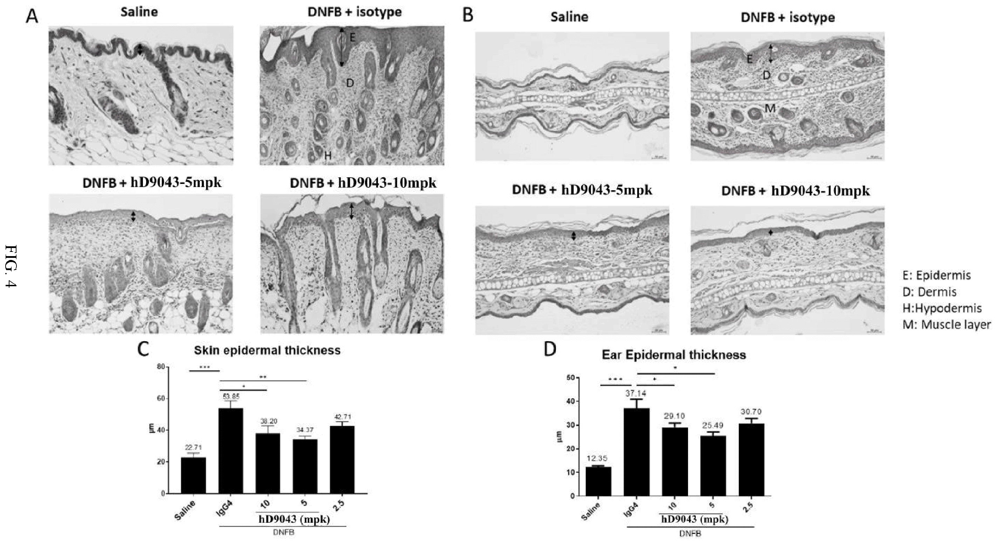

FIG. 4: Anti-IL-17RB antibody hD9043 significantly improved skin health by reducing epidermal thickness. A-B) Representative images taken from paraffin sections of dorsal skin and ears after hematoxylin-eosin staining. Signs of perivasculitis, regular acanthosis and spongiosis elongation of rete ridges were observed in DNFB-treated mice, while anti-IL-17RB antibody hD9043 could suppress these pathologies; C-D) Quantification showing that anti-IL-17RB antibody hD9043 could suppress DNFB-induced epidermal hyperplasia in both dorsal skins and ears. *** p < 0.001 compared with the normal saline control group, as analyzed by Student's t-test. ** p < 0.01, * p < 0.05 compared with the IgG4+DNFB group, as analyzed by one-way ANOVA with post-hoc test. N=8-9.

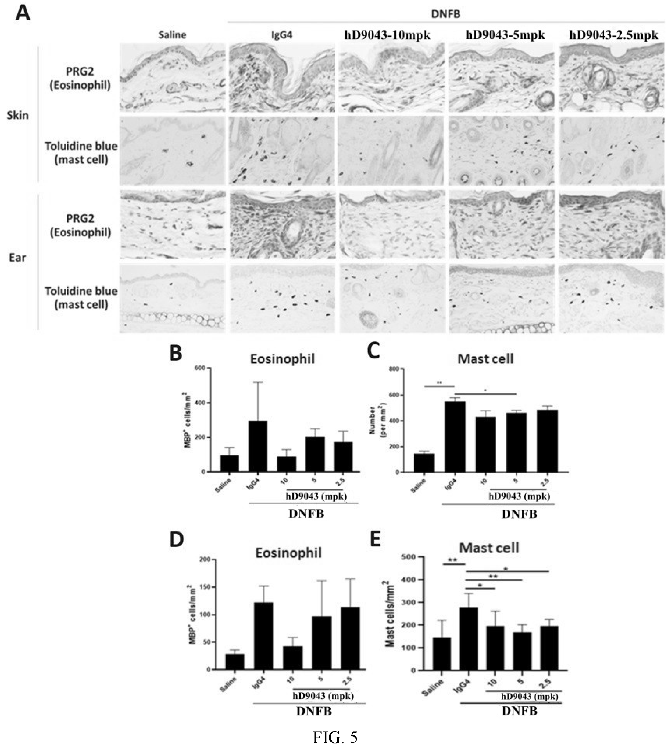

FIG. 5: Anti-IL-17RB antibody hD9043 significantly suppressed the infiltration of eosinophils and mast cells into dorsal skin and ear layers under DNFB sensitization. A) Representative images of eosinophil and mast cell infiltration into dorsal skin and ear layers after DNFB treatment; B-C) Quantification of mast cell number and eosinophil intensity in dorsal skin; D-E) Quantification of mast cell number and eosinophil intensity in ears. ** p < 0.01 compared with the normal saline control group, as analyzed by Student's t-test. ** p < 0.01, * p < 0.05 compared with the IgG4+DNFB group, as analyzed by one-way ANOVA with post-hoc test. N=6.

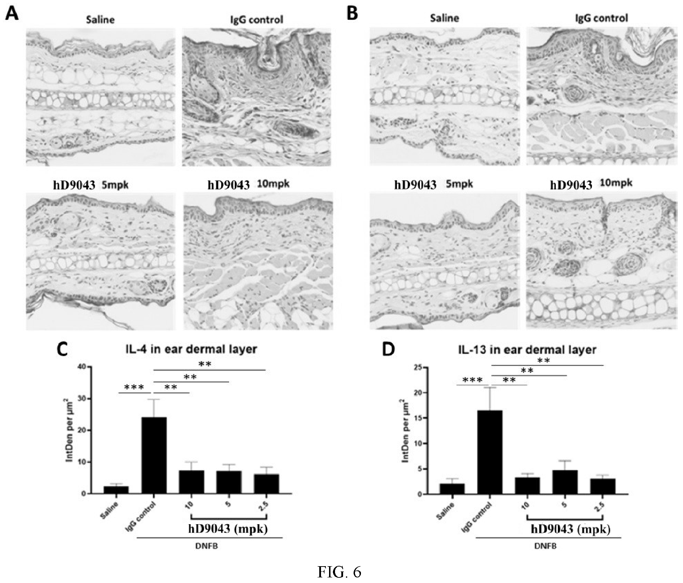

FIG. 6: Anti-IL-17RB antibody hD9043 attenuated Th2 inflammation in ear sections of DNFB-treated animals. A) Representative images of IL-4 expression in different layers; B) Representative images of IL-13 expression in different layers; C-D) Quantification of IL-4 and IL-13 intensities in epidermal layer and dorsal layer. *** p < 0.001 compared with the normal saline control group, as analyzed by Student's t-test. ** p < 0.01 compared with the IgG4+DNFB group, as analyzed by one-way ANOVA with post-hoc test. N=6.

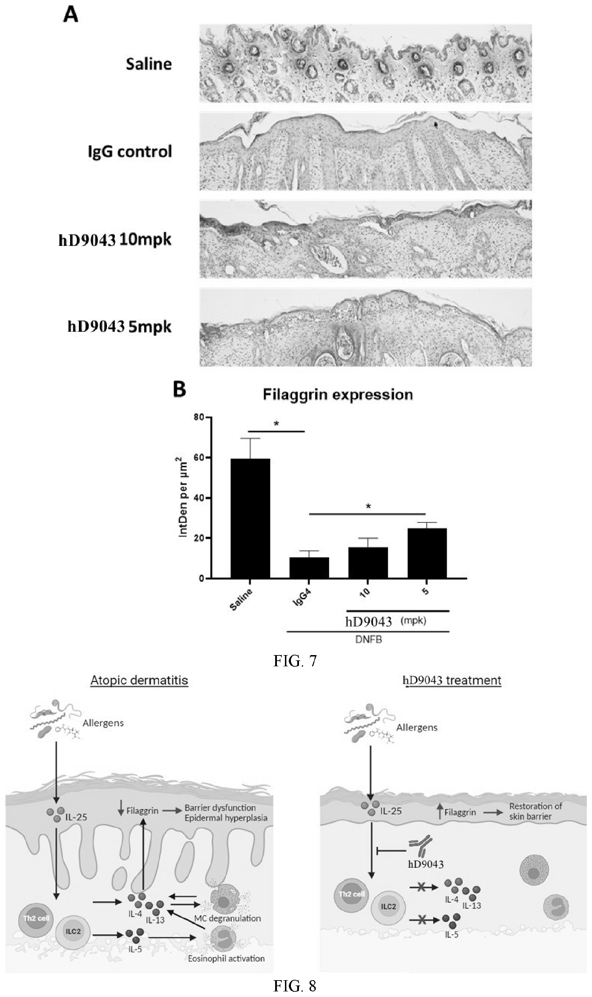

FIG. 7: Anti-IL-17RB antibody hD9043 restored filaggrin expression in DNFB-treated animals. A) Representative images showing that DNFB administration significantly reduced filaggrin levels, while anti-IL-17RB antibody hD9043 could restore its expression; B) Quantification confirming that 5 mg/kg anti-IL-17RB antibody hD9043 could rescue filaggrin levels. * p < 0.05 compared with the normal saline control group, as analyzed by Student's t-test. * p < 0.05 compared with the IgG4+DNFB group, as analyzed by one-way ANOVA with post-hoc test. N=5-6.
FIG. 8: Summary of how administration of anti-IL-17RB antibodies (e.g., hD9043) rescues skin conditions during AD progression.

### EMBODIMENTS OF THE INVENTION

Unless otherwise specified, the following terms and phrases used herein are intended to have the definitions set forth below. A particular term or phrase should not be considered indefinite or unclear in the absence of a specific definition, but should be understood in accordance with the general meaning commonly defined in the art.

The term "antibody" herein refers to any protein or polypeptide capable of specifically recognizing and binding to an antigen, encompassing natural antibodies and artificial antibodies, including but not limited to monoclonal antibodies, polyclonal antibodies, multispecific antibodies, single-chain antibodies, single-domain antibodies, etc., that exhibit the desired biological activities, and can be divided into 5 isotypes according to heavy chain classes, namely IgG, IgM, IgD, IgA and IgE. The term "isolated" means that the target product (e.g., antibody, or antigen-binding fragment) has been separated from its natural environment.

An "antigen-binding fragment" or "antigen-binding portion" of an antibody refers to one or more fragments of an antibody that retain the function of specifically binding to an antigen. Examples of antigen-binding fragments include, but are not limited to, Fab fragments, Fab' fragments, F(ab')2 fragments, Fd fragments, Fv fragments, dAb fragments, isolated CDR regions, single-chain Fv molecules (scFv), and the like.

When referring to an antibody, the term "isolated" herein means that the antibody is substantially free of other cellular components with which it is associated in its natural state. For example, an isolated antibody may be an antibody that has been removed from its natural or native environment.

A "humanized antibody" refers to an antibody that contains complementarity-determining regions (CDRs) derived from non-human animals, as well as framework regions and constant regions derived from humans.

The term "variable region (V)" as used herein refers to regions in antibody light and heavy chains with highly variable amino acid sequences, including light chain variable region (VL) and heavy chain variable region (VH). The term "constant region (C)" refers to regions at the carboxyl terminus of antibody peptides with relatively conserved amino acid sequences in antibody light and heavy chains, including light chain constant region (CL) and heavy chain constant region (CH).

The term "CDR" (complementarity-determining region), also known as "hypervariable region (HVR)", as used herein refers to regions with highly variable sequences in the heavy chain variable region and light chain variable region, which may include 3 CDRs in the heavy chain variable region (i.e., HCDR1 to HCDR3) and 3 CDRs in the light chain variable region (i.e., LCDR1 to LCDR3). The regions other than CDRs in the heavy chain variable region and the light chain variable region are designated as "framework regions (FR)". A variety of numbering systems well known in the art can be used to number the CDRs of heavy and light chains starting from the N-terminus, for example, Kabat, Chothia, Aho, IMGT and Contact (see, Kabat et al., Sequences of Proteins of Immunological Interest, 5th edition, 1991, U.S. Department of Health and Human Services, NIH Publication No. 91-3242; Johnson et al., Nucleic Acids Res 2001, 29:205-206; Chothia & Lesk, J Mol Biol 1987, 196:901-917; Chothia et al., Nature 1989, 342:877-883; Chothia et al., J Mol Biol 1992, 227:799-817; Al-Lazikani et al., J Mol Biol 1997, 273:927-748; Lefranc MP et al., Nucleic Acids Research 1999, 27:209-212; MacCallum RM et al., J Mol Biol 1996, 262:732-745; http://imgt.cines.fr; Dunbar J, Deane CM. ANARCI: antigen receptor numbering and receptor classification. Bioinformatics. 2016; 32(2):298-300).

**Table 1. CDRs determined using different numbering systems**

| CDR | Kabat | AbM | Chothia | Contact | IMGT |
|---|---|---|---|---|---|
| LCDR1 | L24-L34 | L24-L34 | L26-L32 | L30-L36 | L27-L32 |
| LCDR2 | L50-L56 | L50-L56 | L50-L52 | L46-L55 | L50-L52 |
| LCDR3 | L89-L97 | L89-L97 | L91-L96 | L89-L96 | L89-L96 |
| HCDR1 | H31-H35B | H26-H35B | H26-H32 | H30-H35B | H26-H35B |
| HCDR1 | H31-H35 | H26-H35 | H26-H32 | H30-H35 | H26-H35 |
| HCDR2 | H50-H65 | H50-H58 | H53-H55 | H47-H58 | H51-H57 |
| HCDR3 | H95-H102 | H95-H102 | H96-H101 | H93-H101 | H93-H102 |

The "percent identity" of amino acid sequences refers to the percentage of amino acid residues in the candidate sequence that are identical to those in the reference sequence relative to the total number of amino acid residues thereof, after aligning the candidate sequence with the reference sequence and introducing gaps if necessary to achieve the maximum percent sequence identity, without considering any conservative substitutions as part of the sequence identity. Alignment of two or more sequences for determining percent sequence identity of amino acid sequences can be achieved using tools known in the art, such as BLASTp, ClustalW2 (see Higgins DG et al., Methods Enzymol 1996, 266:383-402; Larkin MA et al., Bioinformatics 2007, 23:2947-2948), ALIGN or Megalign (DNASTAR) software, and the like.

The terms "X" and "Xaa" herein are equivalent and refer to an unspecified amino acid, the scope of which is specified by the definition in the relevant expression. To distinguish multiple "X"s in the same amino acid sequence, multiple Xs appearing sequentially are numbered separately (i.e., written as Xn) and their respective scopes are defined separately.

The terms "subject", "patient" and "individual" are used interchangeably herein and include mammals or non-mammalian vertebrates (e.g., chickens, emus, fish); the mammals include, but are not limited to, domesticated animals (e.g., cattle, sheep, cats, dogs, pigs and horses), primates (e.g., humans, non-human primates such as monkeys), rabbits and rodents (e.g., mice, rats, guinea pigs, hamsters), preferably humans.

The term "affinity" or "binding affinity" refers to the intrinsic binding ability of the interaction between members of a binding pair. The affinity between one molecule and another one in a binding pair is typically represented by the equilibrium dissociation constant (K_{D}).

The term "treating", "treatment" or "treat" herein encompasses prophylactic treatment and refers to alleviating, ameliorating, relieving or delaying diseases or related symptoms, reducing or slowing the rate of onset or progression of diseases or related symptoms, reducing the risk of developing diseases or related symptoms, maintaining diseases or related symptoms, achieving a complete or partial reversal of diseases or related symptoms, or curing diseases or related symptoms.

The term "pharmaceutically acceptable" refers to those compounds, materials, compositions and/or dosage forms that, within the scope of sound medical judgment, are suitable for use in contact with the tissues of humans and animals without excessive toxicity, irritation, allergic reactions or other problems or complications, commensurate with a reasonable benefit/risk ratio.

Unless the context clearly indicates otherwise, singular terms encompass plural referents and *vice versa*. Similarly, unless the context clearly indicates otherwise, the word "or" is intended to include "and".

Unless otherwise specified, the terms "comprise", "comprises" and "comprising" or their equivalents (contain, contains, containing, include, includes, including) herein are open-ended mode expressions, meaning that in addition to the listed elements, components and steps, other unspecified elements, components and steps may also be included.

Unless otherwise specified, all numbers expressing amounts of ingredients, measurements or reaction conditions used herein should be understood as being modified in all instances by the term "about". The term "about" herein shall be construed as being within an acceptable error range for the corresponding value. When associated with a percentage, the term "about" may mean, for example, ±1%, preferably ±0.5%, more preferably ±0.1%.

The anti-IL-17RB antibody of the present application is capable of specifically recognizing and binding to human IL-17RB, inhibiting downstream signal transduction of the IL-17RB signaling pathway, reversing the downregulation of FLG expression driven by Th2, and reducing the ILC2 population in lymph nodes.

Next, the technical solutions of the present application will be described in more detail through exemplary embodiments, but the protection scope of the present application is not limited thereto.

### 1. Use

In some embodiments, the present application relates to use of an isolated anti-IL-17RB antibody or antigen-binding fragment thereof, or a pharmaceutical composition comprising the same in the preparation of a medicament for treating FLG-related diseases, wherein the antibody comprises: HCDR1 set forth in SEQ ID NO:1, HCDR2 set forth in SEQ ID NO:2, and HCDR3 set forth in SEQ ID NO:3; and/or LCDR1 set forth in SEQ ID NO:4, LCDR2 set forth in SEQ ID NO:5, and LCDR3 set forth in SEQ ID NO:6.

In some embodiments, the present application relates to an isolated anti-IL-17RB antibody or antigen-binding fragment thereof, or a pharmaceutical composition comprising the same for use in treating FLG-related diseases, wherein the antibody comprises: HCDR1 set forth in SEQ ID NO:1, HCDR2 set forth in SEQ ID NO:2, and HCDR3 set forth in SEQ ID NO:3; and/or LCDR1 set forth in SEQ ID NO:4, LCDR2 set forth in SEQ ID NO:5, and LCDR3 set forth in SEQ ID NO:6.

In some embodiments, the present application relates to a medicament for treating FLG-related diseases, comprising an isolated anti-IL-17RB antibody or antigen-binding fragment thereof, or a pharmaceutical composition coomprising the same, wherein the antibody comprises: HCDR1 set forth in SEQ ID NO:1, HCDR2 set forth in SEQ ID NO:2, and HCDR3 set forth in SEQ ID NO:3; and/or LCDR1 set forth in SEQ ID NO:4, LCDR2 set forth in SEQ ID NO:5, and LCDR3 set forth in SEQ ID NO:6.

In some embodiments, the FLG-related disease is at least one of atopic dermatitis, allergic rhinitis, eczema (e.g., herpetiform eczema herpeticum), contact dermatitis (e.g., allergic contact dermatitis), urticaria, angioedema, psoriasis, dermatitis herpetiformis, bullous pemphigoid, pemphigus, epidermolysis bullosa, rosacea, ichthyosis, solar dermatitis, or related symptoms thereof.

In some embodiments, the FLG-related disease is atopic dermatitis or a related symptom thereof.

In some specific embodiments, the atopic dermatitis is infantile atopic dermatitis, childhood atopic dermatitis, or adolescent and adult atopic dermatitis. In some specific embodiments, the atopic dermatitis is mild, moderate, or severe atopic dermatitis. Various methods for evaluating the severity of atopic dermatitis are known in the art, such as the Scoring Atopic Dermatitis (SCORAD), Eczema Area and Severity Index (EASI), Investigator's Global Assessment (IGA), Visual Analog Scale (VAS) for pruritus severity, etc. Alternatively, simple indicators can also be used clinically to judge the severity of atopic dermatitis, for example: mild atopic dermatitis is defined as a rash area of less than 5%; moderate atopic dermatitis is defined as 5% to 10%, or recurrent rash; and severe atopic dermatitis is defined as skin lesions exceeding 10% of the body surface area, or persistent dermatitis with severe pruritus affecting sleep.

In some specific embodiments, the related symptom of atopic dermatitis comprises dry skin, cutaneous pruritus, eczematous skin lesions (including erythema, papules, redness and swelling, blisters, skin erosion, epidermal thickening), symptoms associated with xeroderma, symptoms related to palmar hyperlinearity, white dermatographia, Hertoghe's sign, infraorbital folds, periorbital darkening (periorbital dark circles), pityriasis alba, and low hairline.

In some embodiments, the anti-IL-17RB antibody comprises: HCDR1 set forth in SEQ ID NO:1, HCDR2 set forth in SEQ ID NO:12 or 13, and HCDR3 set forth in SEQ ID NO:3; and/or LCDR1 set forth in SEQ ID NO:4, LCDR2 set forth in SEQ ID NO:5, and LCDR3 set forth in SEQ ID NO:6.

In some embodiments, the anti-IL-17RB antibody comprises: HCDR1 set forth in SEQ ID NO:1, HCDR2 set forth in SEQ ID NO:12, and HCDR3 set forth in SEQ ID NO:3; and/or LCDR1 set forth in SEQ ID NO:4, LCDR2 set forth in SEQ ID NO:5, and LCDR3 set forth in SEQ ID NO:6.

In some embodiments, the anti-IL-17RB antibody comprises: a VH having at least 80%, at least 85%, at least 90%, at least 91%, at least 92%, at least 93%, at least 94%, at least 95%, at least 96%, at least 97%, at least 98%, at least 99% identity to the amino acid sequence set forth in SEQ ID NO: 7
(X₁VQLVQSGAEVKKPGASVKVSCKX₂SGYTFISYWMNWVRQAPGQGLEWMGRIDPYDSEI QYX₃QKFX₄X₅RVTX₆TRDTSISTAYMELSRLRSDDTAVYYCARSGGFDWFAYWGQGTLVTV

SS); and/or a VL having at least 80%, at least 85%, at least 90%, at least 91%, at least 92%, at least 93%, at least 94%, at least 95%, at least 96%, at least 97%, at least 98%, at least 99% identity to the amino acid sequence set forth in SEQ ID NO: 11.

In some specific embodiments, compared with the amino acid sequence set forth in SEQ ID NO: 7, differing amino acids in an amino acid sequence having at least 80% identity thereto are predominantly (or entirely) present in FR (framework region). In some specific embodiments, compared with the amino acid sequence set forth in SEQ ID NO: 11, differing amino acids in an amino acid sequence having at least 80% identity thereto are predominantly (or entirely) present in FR (framework region).

In some specific embodiments, the VH comprises the amino acid sequence set forth in SEQ ID NO: 7, and optionally the amino acid sequence has at most four (e.g., 1, 2, 3, 4) additional amino acid substitutions in a framework region.

In some specific embodiments, the VH comprises the amino acid sequence set forth in SEQ ID NO: 7, wherein X₃ is N. Further, X₄ is K. Further, X₅ is D. Further, X₁ is Q. Further, X₂ is A. Further, X₆ is M.

In some specific embodiments, the VH comprises the amino acid sequence set forth in SEQ ID NO: 7, wherein X₁ is E. Further, X₂ is T. Further, X₆ is L.

In some specific embodiments, the VH comprises the amino acid sequence set forth in SEQ ID NO: 7, wherein X₃ is A. Further, X₄ is Q. Further, X₅ is G. Further, X₁ is E. Further, X₂ is T. Further, X₆ is L.

In some embodiments, the anti-IL-17RB antibody comprises: a VH having at least 80%, at least 85%, at least 90%, at least 91%, at least 92%, at least 93%, at least 94%, at least 95%, at least 96%, at least 97%, at least 98%, at least 99% identity to the amino acid sequence set forth in any one of SEQ ID NO: 8, 9, or 10; and/or a VL having at least 80%, at least 85%, at least 90%, at least 91%, at least 92%, at least 93%, at least 94%, at least 95%, at least 96%, at least 97%, at least 98%, at least 99% identity to the amino acid sequence set forth in SEQ ID NO:11.

In some specific embodiments, compared with the amino acid sequence set forth in any one of SEQ ID NO: 8, 9, 10 or 11, differing amino acids in an amino acid sequence having at least 80% identity thereto are predominantly (or entirely) present in FR (framework region).

In some embodiments, the anti-IL-17RB antibody comprises: a VH having at least 80%, at least 85%, at least 90%, at least 91%, at least 92%, at least 93%, at least 94%, at least 95%, at least 96%, at least 97%, at least 98%, at least 99% identity to the amino acid sequence set forth in SEQ ID NO: 8; and/or a VL having at least 80%, at least 85%, at least 90%, at least 91%, at least 92%, at least 93%, at least 94%, at least 95%, at least 96%, at least 97%, at least 98%, at least 99% identity to the amino acid sequence set forth in SEQ ID NO:11. Preferably, compared with the amino acid sequence set forth in SEQ ID NO: 8, or 11, differing amino acids in an amino acid sequence having at least 80% identity thereto are predominantly (or entirely) present in FR (framework region).

In some embodiments, the anti-IL-17RB antibody comprises: a VH of the amino acid sequence set forth in SEQ ID NO: 8, and a VL of the amino acid sequence set forth in SEQ ID NO: 11.

In some embodiments, the anti-IL-17RB antibody further comprises: a heavy chain constant region (CH) having at least 80%, at least 85%, at least 90%, at least 91%, at least 92%, at least 93%, at least 94%, at least 95%, at least 96%, at least 97%, at least 98%, at least 99% identity to the amino acid sequence set forth in SEQ ID NO:14 or 18. In some specific embodiments, the anti-IL-17RB antibody further comprises: a CH set forth in SEQ ID NO: 14 or 18. In some specific embodiments, the anti-IL-17RB antibody further comprises a CH set forth in SEQ ID NO: 18.

In some embodiments, the anti-IL-17RB antibody further comprises a light chain constant region (CL), which is selected from the constant regions of a human kappa chain and a human lambda chain, preferably a human kappa chain.

In some embodiments, the anti-IL-17RB antibody further comprises: a CL having at least 80%, at least 85%, at least 90%, at least 91%, at least 92%, at least 93%, at least 94%, at least 95%, at least 96%, at least 97%, at least 98%, at least 99% identity to the amino acid sequence set forth in SEQ ID NO:16. In some specific embodiments, the anti-IL-17RB antibody further comprises a CL set forth in SEQ ID NO:16.

In some embodiments, the anti-IL-17RB antibody comprises: a heavy chain having at least 80%, at least 85%, at least 90%, at least 91%, at least 92%, at least 93%, at least 94%, at least 95%, at least 96%, at least 97%, at least 98%, at least 99% identity to the amino acid sequence set forth in SEQ ID NO: 15, 19, 20 or 21. In some specific embodiments, the anti-IL-17RB antibody comprises a heavy chain set forth in SEQ ID NO:15, 19, 20, or 21. In some specific embodiments, the anti-IL-17RB antibody comprises a heavy chain set forth in SEQ ID NO:19.

In some embodiments, the anti-IL-17RB antibody comprises: a light chain having at least 80%, at least 85%, at least 90%, at least 91%, at least 92%, at least 93%, at least 94%, at least 95%, at least 96%, at least 97%, at least 98%, at least 99% identity to the amino acid sequence set forth in SEQ ID NO:17. In some specific embodiments, the anti-IL-17RB antibody comprises a light chain set forth in SEQ ID NO: 17.

In some embodiments, the anti-IL-17RB antibody is of an IgG1, IgG2, IgG3, or IgG4 isotype, preferably an IgG4 isotype.

In some embodiments, the antigen-binding fragment of the isolated anti-IL-17RB antibody in the present application is selected from a Fab fragment, a Fab' fragment, an F(ab')2 fragment, an Fd fragment, an Fv fragment, a dAb fragment, an isolated CDR region, an scFv, and a nanobody.

Various variants of the aforementioned isolated anti-IL-17RB antibody or antigen-binding fragment thereof herein retain the ability to specifically bind to the antigen IL-17RB.

In some embodiments, the pharmaceutical composition further comprises a pharmaceutically acceptable excipient.

The excipient described herein may be any pharmaceutically acceptable excipients, such as but not limited to a solvent, a propellant, a solubilizer, a co-solvent, an emulsifier, a colorant, a disintegrant, a filler, a lubricant, a wetting agent, an osmotic pressure regulator, a stabilizer, a glidant, a flavoring agent, a preservative, a suspending agent, an antioxidant, a penetration enhancer, a pH regulator, a surfactant, a diluent, and the like. For other available pharmaceutically acceptable excipients, reference may be made to, for example, Handbook of Pharmaceutical Excipients (4th Edition), edited by R.C. Rowe et al., translated by Zheng Junmin, 2005, Chemical Industry Press.

In some embodiments, the pharmaceutical composition further comprises an additional therapeutic agent, including but not limited to an anti-allergic agent, an analgesic agent, an anti-inflammatory agent, an anesthetic agent, and an antipyretic agent and the like. In some embodiments, the pharmaceutical composition further comprises an additional therapeutic agent for atopic dermatitis, such as a glucocorticoid (e.g., hydrocortisone butyrate, mometasone furoate, econazole/triamcinolone acetonide), a calcineurin inhibitor (e.g., tacrolimus, pimecrolimus), an antibiotic (e.g., amoxicillin/clavulanate potassium, levofloxacin hydrochloride), an antipruritic agent (e.g., doxepin hydrochloride, diclofenac diethylamine, ketoprofen), an antihistamine agent (e.g., loratadine, cetirizine hydrochloride, ebastine, chlorpheniramine maleate), a JAK inhibitor (e.g., ruxolitinib, upadacitinib), a PDE4 inhibitor (e.g., crisaborole, roflumilast), calamine lotion, and the like.

In some embodiments, the anti-IL-17RB antibody or antigen-binding fragment thereof, or the pharmaceutical composition comprosing the same described herein may be in the form of a sterile aqueous solution, suspension, emulsion, liposomal formulation or powder. In some embodiments, the anti-IL-17RB antibody or antigen-binding fragment thereof, or the pharmaceutical composition comprising the same described herein may be in a unit dosage form for convenient administration to a patient at a desired dose.

The dosage range of the anti-IL-17RB antibody or antigen-binding fragment thereof, or the pharmaceutical composition comprising the same described herein can be empirically determined by a clinician based on the mode of administration (including administration time, administration interval, administration route), the patient's age, weight, gender or pathological condition, diet, excretion rate and sensitivity to the drug, and the like.

The anti-IL-17RB antibody or antigen-binding fragment thereof, or the pharmaceutical composition comprising the same described herein can be prepared into any dosage form known in the art, such as injection, suspension, solution, powder, emulsion, spray, tablet, pill, capsule, granule, ointment, suppository, gel, and the like.

The anti-IL-17RB antibody or antigen-binding fragment thereof, or the pharmaceutical composition comprising the same described herein may be suitable for intravenous, intramuscular, intraarterial, intraarticular, subcapsular, subarachnoid, intraorbital, intracardiac, subcutaneous, parenteral, intraperitoneal, intraspinal, intranasal or epidermal administration, for example, administration by injection or infusion.

### 2. Therapeutic Method

The present application relates to a method of treating FLG-related diseases, comprising administering to a subject in need thereof an isolated anti-IL-17RB antibody or antigen-binding fragment thereof, or a pharmaceutical composition comprising the same, wherein the antibody comprises: HCDR1 set forth in SEQ ID NO:1, HCDR2 set forth in SEQ ID NO:2, and HCDR3 set forth in SEQ ID NO:3; and/or LCDR1 set forth in SEQ ID NO:4, LCDR2 set forth in SEQ ID NO:5, and LCDR3 set forth in SEQ ID NO:6.

In some embodiments, a therapeutically effective amount or a prophylactically effective amount of the anti-IL-17RB antibody or antigen-binding fragment thereof, or the pharmaceutical composition comprising the same is administered to a subject in need thereof. The therapeutically effective amount or prophylactically effective amount can be routinely or empirically determined by a clinician according to the individual condition of the subject, disease severity, gender, age, weight, administration mode, and the like. In some embodiments, the FLG-related disease is at least one of atopic dermatitis, allergic rhinitis, eczema (e.g., eczema herpeticum), contact dermatitis (e.g., allergic contact dermatitis), urticaria, angioedema, psoriasis, dermatitis herpetiformis, bullous pemphigoid, pemphigus, epidermolysis bullosa, rosacea, ichthyosis, solar dermatitis, or related symptoms thereof.

In some embodiments, the FLG-related disease is atopic dermatitis or a related symptom thereof.

In some specific embodiments, the atopic dermatitis is infantile atopic dermatitis, childhood atopic dermatitis, or adolescent and adult atopic dermatitis. In some specific embodiments, the atopic dermatitis is mild, moderate, or severe atopic dermatitis. In some specific embodiments, the related symptom of atopic dermatitis comprises dry skin, cutaneous pruritus, eczematous skin lesions (including erythema, papules, redness and swelling, blisters, skin erosion, epidermal thickening), symptoms associated with xeroderma, symptoms related to palmar hyperlinearity, white dermatographia, Hertoghe's sign, infraorbital folds, periorbital darkening (periorbital dark circles), pityriasis alba, and low hairline.

In some embodiments, the anti-IL-17RB antibody comprises: HCDR1 set forth in SEQ ID NO:1, HCDR2 set forth in SEQ ID NO:12 or 13, and HCDR3 set forth in SEQ ID NO:3; and/or LCDR1 set forth in SEQ ID NO:4, LCDR2 set forth in SEQ ID NO:5, and LCDR3 set forth in SEQ ID NO:6.

In some embodiments, the anti-IL-17RB antibody comprises: HCDR1 set forth in SEQ ID NO:1, HCDR2 set forth in SEQ ID NO:12, and HCDR3 set forth in SEQ ID NO:3; and/or LCDR1 set forth in SEQ ID NO:4, LCDR2 set forth in SEQ ID NO:5, and LCDR3 set forth in SEQ ID NO:6.

In some embodiments, the anti-IL-17RB antibody comprises: a VH having at least 80%, at least 85%, at least 90%, at least 91%, at least 92%, at least 93%, at least 94%, at least 95%, at least 96%, at least 97%, at least 98%, at least 99% identity to the amino acid sequence set forth in SEQ ID NO: 7; and/or a VL having at least 80%, at least 85%, at least 90%, at least 91%, at least 92%, at least 93%, at least 94%, at least 95%, at least 96%, at least 97%, at least 98%, at least 99% identity to the amino acid sequence set forth in SEQ ID NO:11.

In some specific embodiments, compared with the amino acid sequence set forth in SEQ ID NO: 7, differing amino acids in an amino acid sequence having at least 80% identity thereto are predominantly (or entirely) present in FR (framework region). In some specific embodiments, compared with the amino acid sequence set forth in SEQ ID NO: 11, differing amino acids in an amino acid sequence having at least 80% identity thereto are predominantly (or entirely) present in FR (framework region).

In some specific embodiments, the VH comprises the amino acid sequence set forth in SEQ ID NO: 7, and optionally the amino acid sequence has at most four additional amino acid substitutions in a framework region.

In some specific embodiments, the VH comprises the amino acid sequence set forth in SEQ ID NO: 7, wherein X₃ is N. Further, X₄ is K. Further, X₅ is D. Further, X₁ is Q. Further, X₂ is A. Further, X₆ is M.

In some specific embodiments, the VH comprises the amino acid sequence set forth in SEQ ID NO: 7, wherein X₁ is E. Further, X₂ is T. Further, X₆ is L.

In some specific embodiments, the VH comprises the amino acid sequence set forth in SEQ ID NO: 7, wherein X ₃ is A. Further, X₄ is Q. Further, X₅ is G. Further, X₁ is E. Further, X₂ is T. Further, X₆ is L.

In some embodiments, the anti-IL-17RB antibody comprises a VH having at least 80%, at least 85%, at least 90%, at least 91%, at least 92%, at least 93%, at least 94%, at least 95%, at least 96%, at least 97%, at least 98%, at least 99% identity to the amino acid sequence set forth in any one of SEQ ID NO: 8, 9, or 10; and/or a VL having at least 80%, at least 85%, at least 90%, at least 91%, at least 92%, at least 93%, at least 94%, at least 95%, at least 96%, at least 97%, at least 98%, at least 99% identity to the amino acid sequence set forth in SEQ ID NO:11.

In some specific embodiments, compared with the amino acid sequence set forth in any one of SEQ ID NO: 8, 9, 10 or 11, differing amino acids in an amino acid sequence having at least 80% identity relative to the amino acid sequence thereto are predominantly (or entirely) present in FR (framework region).

In some embodiments, the anti-IL-17RB antibody comprises: a VH having at least 80%, at least 85%, at least 90%, at least 91%, at least 92%, at least 93%, at least 94%, at least 95%, at least 96%, at least 97%, at least 98%, at least 99% identity to the amino acid sequence set forth in SEQ ID NO: 8; and/or a VL having at least 80%, at least 85%, at least 90%, at least 91%, at least 92%, at least 93%, at least 94%, at least 95%, at least 96%, at least 97%, at least 98%, at least 99% identity to the amino acid sequence set forth in SEQ ID NO:11. Preferably, compared with the amino acid sequence set forth in SEQ ID NO: 8, or 11, differing amino acids in an amino acid sequence having at least 80% identity thereto are predominantly (or entirely) present in FR (framework region).

In some embodiments, the anti-IL-17RB antibody comprises: a VH of the amino acid sequence set forth in SEQ ID NO: 8, and a VL of the amino acid sequence set forth in SEQ ID NO: 11.

In some embodiments, the anti-IL-17RB antibody further comprises: a heavy chain constant region (CH) having at least 80%, at least 85%, at least 90%, at least 91%, at least 92%, at least 93%, at least 94%, at least 95%, at least 96%, at least 97%, at least 98%, at least 99% identity to the amino acid sequence set forth in SEQ ID NO:14 or 18. In some specific embodiments, the anti-IL-17RB antibody further comprises: a CH set forth in SEQ ID NO: 14 or 18. In some specific embodiments, the anti-IL-17RB antibody further comprises a CH set forth in SEQ ID NO:18.

In some embodiments, the anti-IL-17RB antibody further comprises a light chain constant region (CL), which is selected from the constant regions of a human kappa chain and a human lambda chain, preferably a human kappa chain.

In some embodiments, the anti-IL-17RB antibody further comprises: a CL having at least 80%, at least 85%, at least 90%, at least 91%, at least 92%, at least 93%, at least 94%, at least 95%, at least 96%, at least 97%, at least 98%, at least 99% identity to the amino acid sequence set forth in SEQ ID NO:16. In some specific embodiments, the anti-IL-17RB antibody further comprises a CL set forth in SEQ ID NO:16.

In some embodiments, the anti-IL-17RB antibody comprises a heavy chain having at least 80%, at least 85%, at least 90%, at least 91%, at least 92%, at least 93%, at least 94%, at least 95%, at least 96%, at least 97%, at least 98%, at least 99% identity to the amino acid sequence set forth in SEQ ID NO:15, 19, 20 or 21. In some specific embodiments, the anti-IL-17RB antibody comprises a heavy chain set forth in SEQ ID NO:15, 19, 20, or 21. In some specific embodiments, the anti-IL-17RB antibody comprises a heavy chain set forth in SEQ ID NO:19.

In some embodiments, the anti-IL-17RB antibody comprises a light chain having at least 80%, at least 85%, at least 90%, at least 91%, at least 92%, at least 93%, at least 94%, at least 95%, at least 96%, at least 97%, at least 98%, at least 99% identity to the amino acid sequence set forth in SEQ ID NO:17. In some specific embodiments, the anti-IL-17RB antibody comprises a light chain set forth in SEQ ID NO: 17.

In some embodiments, the anti-IL-17RB antibody is of an IgG1, IgG2, IgG3, or IgG4 isotype, preferably an IgG4 isotype.

In some embodiments, the antigen-binding fragment of the isolated anti-IL-17RB antibody in the present application is selected from a Fab fragment, a Fab' fragment, an F(ab')2 fragment, an Fd fragment, an Fv fragment, a dAb fragment, an isolated CDR region, an scFv, and a nanobody.

Various variants of the aforementioned isolated anti-IL-17RB antibody or antigen-binding fragment thereof herein retain the ability to specifically bind to the antigen IL-17RB.

In some embodiments, the pharmaceutical composition further comprises a pharmaceutically acceptable excipient.

The excipient described herein may be any pharmaceutically acceptable excipients, such as but not limited to a solvent, a propellant, a solubilizer, a co-solvent, an emulsifier, a colorant, a disintegrant, a filler, a lubricant, a wetting agent, an osmotic pressure regulator, a stabilizer, a glidant, a flavoring agent, a preservative, a suspending agent, an antioxidant, a penetration enhancer, a pH regulator, a surfactant, a diluent, and the like. For other available pharmaceutically acceptable excipients, reference may be made to, for example, Handbook of Pharmaceutical Excipients (4th Edition), edited by R.C. Rowe et al., translated by Zheng Junmin, 2005, Chemical Industry Press.

In some embodiments, the pharmaceutical composition further comprises an additional therapeutic agent, including but not limited to an anti-allergic agent, an analgesic agent, an anti-inflammatory agent, an anesthetic agent, and an antipyretic agent and the like. In some embodiments, the pharmaceutical composition further comprises an additional therapeutic agent for atopic dermatitis, such as a glucocorticoid (e.g., hydrocortisone butyrate, mometasone furoate, econazole/triamcinolone acetonide), a calcineurin inhibitor (e.g., tacrolimus, pimecrolimus), an antibiotic (e.g., amoxicillin/clavulanate potassium, levofloxacin hydrochloride), an antipruritic agent (e.g., doxepin hydrochloride, diclofenac diethylamine, ketoprofen), an antihistamine agent (e.g., loratadine, cetirizine hydrochloride, ebastine, chlorpheniramine maleate), a JAK inhibitor (e.g., ruxolitinib, upadacitinib), a PDE4 inhibitor (e.g., crisaborole, roflumilast), calamine lotion, and the like.

In certain embodiments, the subject is selected from humans, non-human primates, or murines, preferably humans.

### Examples

The present application is described in detail below by way of examples, but it does not mean that the protection scope of the present application is only limited thereto. The present application has been described in detail herein, and specific embodiments thereof have also been disclosed. It will be apparent to those skilled in the art that various modifications and improvements to the specific embodiments of the present application can be made without departing from the spirit and scope of the present application.

Unless otherwise specified, all reagents, materials, instruments, and the like involved in the following examples are known in the art and are commercially available.

### Preparation Example 1 Preparation of anti-IL-17RB antibodies

Humanized anti-IL-17RB antibodies hD9040, hD9041 and hD9042 were prepared according to the method disclosed in the "Experiments" section of WO2020115319A1, and IgG4 antibody hD9043 was prepared according to the method described therein.

By ELISA test, the humanized antibodies had similar EC₅₀ values for binding to human IL-17RB; by Biacore assay, the humanized antibodies had good binding affinity to human IL-17RB. The humanized antibodies also exhibited good high-temperature thermal stability, no aggregation problems, low non-specific interaction tendency and good solubility.

**Table 2. Amino acid sequences of anti-IL-17RB antibodies**

| | hD9040 | hD9041 | hD9042 | hD9043 (IgG4) |
|---|---|---|---|---|
| HCDR 1 | SYWMN (SEQ ID NO:1) | SYWMN (SEQ ID NO:1) | SYWMN (SEQ ID NO:1) | SYWMN (SEQ ID NO:1) |
| HCDR 2 | RIDPYDSEIQYN QKFKD (SEQ ID NO:12) | RIDPYDSEIQYA QKFQG (SEQ ID NO:13) | RIDPYDSEIQYN QKFKD (SEQ ID NO:12) | RIDPYDSEIQYNQKF KD (SEQ ID NO:12) |
| HCDR | SGGFDWFAY | SGGFDWFAY | SGGFDWFAY | SGGFDWFAY (SEQ |
| 3 | (SEQ ID NO:3) | (SEQ ID NO:3) | (SEQ ID NO:3) | ID NO:3) |
| VH | EVQLVQSGAEV KKPGASVKVSC KTSGYTFISYWM NWVRQAPGQGL EWMGRIDPYDS EIQYNQKFKDRV TLTRDTSISTAYM ELSRLRSDDTAV YYCARSGGFDW FAYWGQGTLVT VSS (SEQ ID NO:9) | EVQLVQSGAEV KKPGASVKVSC KTSGYTFISYWM NWVRQAPGQGL EWMGRIDPYDS EIQYAQKFQGRV TLTRDTSISTAY MELSRLRSDDTA VYYCARSGGFD WFAYWGQGTLV TVSS (SEQ ID NO:10) | QVQLVQSGAEV KKPGASVKVSC KASGYTFISYW MNWVRQAPGQ GLEWMGRIDPY DSEIQYNQKFKD RVTMTRDTSIST AYMELSRLRSD DTAVYYCARSG GFDWFAYWGQG TLVTVSS (SEQ ID NO:8) | QVQLVQSGAEVKKP GASVKVSCKASGYT FISYWMNWVRQAP GQGLEWMGRIDPYD SEIQYNQKFKDRVT MTRDTSISTAYMELS RLRSDDTAVYYCAR SGGFDWFAYWGQG TLVTVSS (SEQ ID NO:8) |
| LCDR 1 | RASENINSNLA (SEQ ID NO:4) | RASENINSNLA (SEQ ID NO:4) | RASENINSNLA (SEQ ID NO:4) | RASENINSNLA (SEQ ID NO:4) |
| LCDR 2 | DVTNLAD (SEQ ID NO:5) | DVTNLAD (SEQ ID NO:5) | DVTNLAD (SEQ ID NO:5) | DVTNLAD (SEQ ID NO:5) |
| LCDR 3 | QHFWGPPYT (SEQ ID NO:6) | QHFWGPPYT (SEQ ID NO:6) | QHFWGPPYT (SEQ ID NO:6) | QHFWGPPYT (SEQ ID NO:6) |
| VL | DIQMTQSPSSLS ASVGDRVTITCR ASENINSNLAWY QQKPGKAPKLLL YDVTNLADGVP SRFSGSGSGTDY TLTISSLQPEDFA TYYCQHFWGPP YTFGGGTKVEIK (SEQ ID NO:11) | DIQMTQSPSSLS ASVGDRVTITCR ASENINSNLAWY QQKPGKAPKLL LYDVTNLADGV PSRFSGSGSGTD YTLTISSLQPEDF ATYYCQHFWGP PYTFGGGTKVEI K (SEQ ID NO:11) | DIQMTQSPSSLS ASVGDRVTITCR ASENINSNLAWY QQKPGKAPKLL LYDVTNLADGV PSRFSGSGSGTD YTLTISSLQPEDF ATYYCQHFWGP PYTFGGGTKVEI K (SEQ ID NO:11) | DIQMTQSPSSLSASV GDRVTITCRASENIN SNLAWYQQKPGKAP KLLLYDVTNLADGV PSRFSGSGSGTDYTL TISSLQPEDFATYYC QHFWGPPYTFGGGT KVEIK (SEQ ID NO:11) |
| CH | ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPELLG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALPAPIEKTISKAKGQPREPQVYTLPP SREEMTKNQVSLTCLVKGFYPSDIAVEWESNGQPENNYK TTPPVLDSDGSFFLYSKLTVDKSRWQQGNVFSCSVMHEA LHNHYTQKSLSLSPGK (SEQ ID NO:14) | | | ASTKGPSVFPLAPCS RSTSESTAALGCLVK DYFPEPVTVSWNSG ALTSGVHTFPAVLQS SGLYSLSSVVTVPSS SLGTKTYTCNVDHK PSNTKVDKRVESKY GPPCPPCPAPEFLGG PSVFLFPPKPKDTLM ISRTPEVTCVVVDVS QEDPEVQFNWYVD GVEVHNAKTKPREE QFNSTYRVVSVLTVL HQDWLNGKEYKCK VSNKGLPSSIEKTISK AKGQPREPQVYTLP PSQEEMTKNQVSLT CLVKGFYPSDIAVEW ESNGQPENNYKTTPP VLDSDGSFFLYSRLT VDKSRWQEGNVFSC SVMHEALHNHYTQ |
| | | | | KSLSLSLGK (SEQ ID NO:18) |
| CL |  (SEQ ID NO:16) | | | |

Unless otherwise specified, the anti-IL-17RB antibody used in the following examples is the humanized anti-IL-17RB antibody hD9043 prepared above.

### Example 1 Effect of Anti-IL-17RB Antibody on Restoration of Filaggrin Levels In Vitro

Filaggrin is an important protein for the development and maintenance of the skin barrier to protect the host from pathogen, allergen, and ultraviolet-induced damage. Clinical studies have clearly demonstrated that the clinical severity is correlated with barrier impairment in filaggrinrelated eczema AD (Hasebe et al., J Invest Dermatol., 2009; 129(3): 682-9). Filaggrin levels are downregulated in both uninvolved and lesioned skins of AD (Howell et al., J Allergy Clin Immunol., 2007; 120(1): 150-155). In addition, null mutations in *FLG* (the gene encoding filaggrin) are strong genetic factors for AD. *FLG* null mutations have been shown to be correlated with AD severity and persistence into adulthood (Moosbrugger-Martinz et al., Int J Mol Sci., 2022; 23(10):5318).

During the activation of T helper 2 (Th2) immune responses, Th2 cytokines are secreted from Th2-related immune cells to suppress filaggrin expression in keratinocytes, as shown both *in vitro* (Howell et al., J Allergy Clin Immunol., 2007; 120(1): 150-155; Howell et al., J Invest Dermatol., 2008; 128(9): 2248-58) and *in vivo* (Hönzke et al., J Invest Dermatol., 2016; 136(3): 631-639). Since anti-IL-17RB antibodies have previously been shown to exhibit anti-Th2 immunity function in PBMC cultures, the potency of anti-IL-17RB antibodies in reversing Th2-driven filaggrin downregulation needs to be tested.

HaCaT cells, which are immortalized human keratinocytes, were used to test the above phenomenon *in vitro*. HaCaT cells were first terminally differentiated into mature keratinocytes and maintained in the culture medium containing 1.8 mM calcium chloride. Proof-of-concept (POC) studies first demonstrated that direct administration of 50 µg/mL house dust mite (HDM) extract (Jeon et al., Front Immunol., 2021 Oct 8:12:646316) and IL-4/IL-13 cocktail (50 ng/mL) on mature HaCaT cells resulted in a significant reduction in filaggrin expression after 24 hours through Western blot analysis (FIG. 1C). These data validated *in vitro* system to study keratinocyte homeostasis in our lab.

Since IL-25 had no effect on filaggrin levels in mature HaCaT cells, a co-culture system was developed to study how anti-IL-17RB antibodies could modulate filaggrin expression in mature HaCaT cells through PBMCs. Briefly, transwells coated with PBMCs were placed on top of each 12-well plate coated with mature HaCaT cells. Total alarmins (IL-25+IL-33+TSLP, each 20 ng/mL) and the antibodies were administered to the PBMCs and co-cultured with the bottom HaCaT cells for 6 days. Proteins were harvested from HaCaT cells for Western blot analysis. The results indicated that anti-IL-17RB antibodies could restore filaggrin levels in HaCaT cells in a dose-dependent manner (FIGS. 1D- 1E), demonstrating the attenuation of Th2 immunity driven filaggrin downregulation in keratinocytes.

### Example 2 Development of animal models for AD studies

To study the *in vivo* efficacy of anti-IL-17RB antibodies, female BALB/c mice were topically painted with 1-fluoro-2,4-dinitrobenzene (DNFB), a hapten to induce contact dermatitis (Feng et al., Front Med (Lausanne)., 2022:9:843230). Briefly, 0.15% DNFB was prepared in a 3:1 mixture of acetone and olive oil before topical application. After anesthesia, the dorsal skin of the mice was shaved and tape-stripped to disrupt the skin barrier. The dorsal skin (1 cm × 1 cm) and ears of the mice were paintied with 0.15% DNFB once per week for 4 weeks, as shown in FIG. 2A. Antibodies (anti-IL-17RB antibody or isotype IgG4 control) were intraperitoneally injected into mice of respective groups in the same dosing paradigm as DNFB. Tissues were harvested from mice 24 hours after the last antibody treatment. Meanwhile, a healthy mouse control group (also referred to as the non-disease saline group) was established, and normal saline (100 µL) was intraperitoneally injected into the mice in the same dosing paradigm as DNFB.

Dermatitis score was given to each mouse according to the degree of erythema, edema, dryness/scarring, and excoriation/erosion condition observed on the dorsal skin. Each symptom was scored on a scale of 0 (absent), 1 (mild), 2 (moderate), or 3 (severe). The total dermatitis score was the sum of these individual scores (Feng et al., Front Med (Lausanne)., 2022:9:843230). The data demonstrated that the peak of dermatitis was observed on Day 14, and mice were recovered on Day 21, as demonstrated by reduced inflammation and erosion as well as increased hair regrowth (FIG. 2B). Quantification of dermatitis scores confirmed that anti-IL-17RB antibodies at all doses could significantly ameliorate skin conditions compared with the IgG4 control group (FIG. 2C). No dermatitis symptoms were observed in any mice of the non-disease saline group.

### Example 3 Supression of Th2 Immune Response by Anti-IL-17RB Antibody in AD

The global modulation of immune responses was investigated in submental lymph nodes extracted from mice after blood collection. DNFB could significantly lead to lymph node swelling after a month, which could be partially alleviated by anti-IL-17RB antibody (FIG. 3A). Single cells were isolated from lymph nodes via trypsin digestion and seeded into 48-well plates for DNFB restimulation assay for 1 day. Supernatants were collected for ELISA analyses, and the remaining cells were fixed with 4% paraformaldehyde for flow cytometry analyses. Type 2 innate lymphoid cells (ILC2s) are one of the major cell types to drive Th2 immune responses during AD (Salimi et al., J Exp Med., 2013; 210(13): 2939-50). The ILC2 population was gated as Lin⁻/CD45⁺/ST2⁺/ICOS⁺ cells. The results indicated that anti-IL-17RB antibody exhibited an obvious trend to reduce the ILC2 population in submental lymph nodes (FIG. 3B). Consistent with the ILC2 results, IL-4, IL-5 and IL-13 secretions from lymph node cells were all attenuated in the anti-IL-17RB antibody-treated group after DNFB restimulation for 1 day (FIGS. 3C-3E).

### Example 4 Ameliorations of Skin and Ear Pathologies Following Administration of Anti-IL-17RB Antibody

Histological studies in the prepared ear and skin paraffin sections further demonstrated the efficacy of anti-IL-17RB antibody to reduce DNFB-induced dermatitis. Briefly, ear and dorsal skin were isolated from animals after blood collection, fixed in 4% paraformaldehyde, dehydrated in increasing concentration of ethanol, and embedded in paraffin wax. Five-micrometer sections were cut from the tissue blocks by microtome and mounted on pre-coated glass slides.

Hematoxylin-eosin staining was performed on the tissue sections to evaluate structural changes in skin and ear lays following DNFB and anti-IL-17RB antibody treatment. Sections were deparaffinized in two changes of xylene and rehydrated decreasing concentrations of ethanol. After washing in water several times, sections were stained in hematoxylin solution for 1-2 minutes, washed in water to remove excessive stain, and incubated in Scott's tap water for 1 minute. Sections were dehydrated, stained in eosin solution for 1-2 minutes, and washed in 100% ethanol to remove excessive stain. Stained sections were cleaned twice in xylene and mounted with DPX mounting medium.

Impairment in skin barrier function and epidermal thickening are usually initiated by Th2 inflammation and used as indicators for evaluating the severity of AD (Kim et al., Allergy Asthma Proc., 2019; 40(2): 84-92). Epidermal thickness was calculated in random regions in each image for quantification, with 3-4 images being analyzed for each single animal. Representative images demonstrated that one month of DNFB treatment altered both dorsal skin and ear layers in terms of increased epidermal and dorsal thickness, signs of perivasculitis, regular acanthosis, and spongiosis elongation of rete ridges, while the anti-IL-17RB antibody could ameliorate these symptoms (FIGS. 4A-4B). Quantification further confirmed that the anti-IL-17RB antibody could significantly reduce DNFB triggered epidermal hyperplasia in both dorsal skin and ear layers (FIGS. 4C-4D), indicating the reduction in disease severity upon the treatment with the anti-IL-17RB antibody.

### Example 5 Suppression of Immune Cell Infiltration Following Administration of Anti-IL-17RB Antibody

Eosinophils and mast cells are two important cell types involved in the initiation and progression of AD (Kawakami et al., Curr Opin Immunol., 2009; 21(6): 666-678; Radonjic-Hoesli et al., Semin Immunopathol., 2021; 43(3): 393-409). Eosinophils and mast cells could form a positive loop to further enhance Th2 and Th17 activation, leading to the shift of acute to chronic phase of AD. Therefore, the effects of the anti-IL-17RB antibody in mast cells and eosinophils need to be elucidated.

To study the mast cell infiltration, ear and dorsal skin sections were stained with 0.1% toluidine blue for 2-3 minutes to label mast cells, and mounted in DPX mounting medium for microscopic analyses. Mast cells were labelled as pale to dark purple cells under microscopy.

To study the eosinophil infiltration, immunohistochemistry was performed on ear and dorsal skin sections. Briefly, the sections were quenched in 3% hydrogen peroxide after deparaffinization and rehydration. After washing in PBS several times, sections were blocked in 8% FBS diluted in 0.3% triton X-100/PBS for 1 hour at room temperature. Anti-PRG2 primary antibody was diluted in the same blocking buffer and used to incubate the sections at 4°C overnight. Next day, sections were washed and stained with HRP-conjugated secondary antibody for 1 hour at room temperature. Eosinophils were visualized in brown color after application of DAB substrate and mounted on the glass slides using DPX mounting medium. Quantification was performed in Image J software.

Representative images indicated that DNFB could significantly induce infiltration of mast cells and eosinophils into both dorsal skin and ear layers, in which the anti-IL-17RB antibody could suppress both phenomenon in a dose dependent manne (FIG. 5A). Quantification of mast cell number and intensities of eosinophil signals further confirmed the observation in both dorsal skin (FIGS. 5B-5C) and ear (FIGS. 5D-5E).

### Example 6 Filaggrin Restoration Caused by Suppression in Th2 Inflammation via Anti-IL-17RB Antibody

As previously described, Th2 cell expansion leads to Th2 inflammation through increased secretion of Th2 cytokines, such as IL-4 and IL-13, which eventually causing the downregulation of filaggrin level in the skin layers. To confirm this statement in our model, tissue sections were stained with anti-IL-4, anti-IL-13 and anti-filaggrin antisera through immunohistochemistry again.

The positive signals of IL-4 and IL-13 were identified in the epidermal and dorsal layers. Consistent with the results from submental lymph node cultures, DNFB could exaggerate the secretion of IL-4 and IL-3, while anti-IL-17RB antibody could in turm suppress their expressions in the ears (FIG. 6).

Filaggrin expression was also visualized and quantified in the epidermal and dorsal layers. Consistent with the reduction in Th2 inflammation in the skin layers, the anti-IL-17RB antibody could restore the filaggrin level, and potentially ameliorate the skin condition in DNFB-treated mice (FIG. 7).

Overall, the beneficial effects of anti-IL-17RB antibodies have been summarized in FIG. 8. Anti-IL-17RB antibodies could restore the skin barrier and reduce epidermal hyperplasia through suppression of Th2 immune responses, reduction in eosinophil/mast cell infiltration, and restoration of the expression of filaggrin in the skin layers.

All patents, patent applications and other publications are expressly incorporated herein by reference in their entirety for the purpose of description and disclosure. These publications are provided only becasue their publication date precedes the filing date of the present application. All statements regarding the dates of these documents or the representation of the contents of these documents are based on information available to applicants and do not constitute any admission to the correctness of the dates of these documents or the contents of these documents.

Those skilled in the art may make various combinations, modifications, or reconstructions of the embodiments of the present application within the scope and spirit of the present application, and the resulting solutions also fall within the protection scope of the present application.

## Claims

1. Use of an isolated anti-IL-17RB antibody or antigen-binding fragment thereof, or a pharmaceutical composition coomprising the same in the preparation of a medicament for treating FLG-related diseases, wherein the antibody comprises:
HCDR1 set forth in SEQ ID NO: 1, HCDR2 set forth in SEQ ID NO: 2, and HCDR3 set forth in SEQ ID NO: 3; and/or
LCDR1 set forth in SEQ ID NO: 4, LCDR2 set forth in SEQ ID NO: 5, and LCDR3 set forth in SEQ ID NO: 6.

2. The use according to claim 1, wherein the FLG-related disease is at least one of atopic dermatitis, allergic rhinitis, eczema, contact dermatitis, urticaria, angioedema, psoriasis, dermatitis herpetiformis, bullous pemphigoid, pemphigus, epidermolysis bullosa, rosacea, ichthyosis, solar dermatitis, or related symptoms thereof.

3. The use according to claim 1 or 2, wherein the FLG-related disease is atopic dermatitis or a related symptom thereof.

4. The use according to claim 2 or 3, wherein the atopic dermatitis is infantile atopic dermatitis, childhood atopic dermatitis, or adolescent and adult atopic dermatitis.

5. The use according to any one of claims 2 to 4, wherein the atopic dermatitis is mild, moderate, or severe atopic dermatitis.

6. The use according to any one of claims 2 to 5, wherein the related symptom of atopic dermatitis comprises dry skin, cutaneous pruritus, eczematous skin lesions, symptoms associated with xeroderma, symptoms related to palmar hyperlinearity, white dermatographia, Hertoghe's sign, infraorbital folds, periorbital darkening (periorbital dark circles), pityriasis alba, and low hairline.

7. The use according to any one of claims 1 to 6, wherein the anti-IL-17RB antibody comprises:
HCDR1 set forth in SEQ ID NO: 1, HCDR2 set forth in SEQ ID NO: 12 or 13, and HCDR3 set forth in SEQ ID NO: 3; and
LCDR1 set forth in SEQ ID NO: 4, LCDR2 set forth in SEQ ID NO: 5, and LCDR3 set forth in SEQ ID NO: 6.

8. The use according to any one of claims 1 to 7, wherein the anti-IL-17RB antibody comprises:
HCDR1 set forth in SEQ ID NO: 1, HCDR2 set forth in SEQ ID NO: 12, and HCDR3 set forth in SEQ ID NO: 3; and
LCDR1 set forth in SEQ ID NO: 4, LCDR2 set forth in SEQ ID NO: 5, and LCDR3 set forth in SEQ ID NO: 6.

9. The use according to any one of claims 1 to 8, wherein the anti-IL-17RB antibody comprises:
a VH having at least 80% identity to the amino acid sequence set forth in SEQ ID NO: 7; and/or
a VL having at least 80% identity to the amino acid sequence set forth in SEQ ID NO: 11.

10. The use according to claim 9, wherein compared with the amino acid sequence set forth in SEQ ID NO: 7, differing amino acids in an amino acid sequence having at least 80% identity thereto are predominantly or entirely present in FR; and/or compared with the amino acid sequence set forth in SEQ ID NO: 11, differing amino acids in an amino acid sequence having at least 80% identity thereto are predominantly or entirely present in FR.

11. The use according to any one of claims 1 to 8, wherein the VH comprises the amino acid sequence set forth in SEQ ID NO: 7, and optionally the amino acid sequence has at most four additional amino acid substitutions in a framework region.

12. The use according to claim 11, wherein the VH comprises the amino acid sequence set forth in SEQ ID NO: 7, wherein X₃ is N, X₄ is K, X₅ is D, X₁ is Q, X₂ is A, and/or X₆ is M.

13. The use according to claim 11, wherein the VH comprises the amino acid sequence set forth in SEQ ID NO: 7, wherein X₁ is E, X₂ is T, and/or X₆ is L.

14. The use according to claim 11, wherein the VH comprises the amino acid sequence set forth in SEQ ID NO: 7, wherein X₃ is A, X₄ is Q, X₅ is G, X₁ is E, X₂ is T, and/or X₆ is L.

15. The use according to any one of claims 1 to 10, wherein the anti-IL-17RB antibody comprises:
a VH having at least 80% identity to the amino acid sequence set forth in any one of SEQ ID NO: 8, 9, or 10; and
a VL having at least 80% identity to the amino acid sequence set forth in SEQ ID NO: 11.

16. The use according to claim 15, wherein compared with the amino acid sequence set forth in any one of SEQ ID NO: 8, 9, 10 or 11, differing amino acids in an amino acid sequence having at least 80% identity thereto are predominantly or entirely present in FR.

17. The use according to claim 15 or 16, wherein the anti-IL-17RB antibody comprises:
a VH having at least 80% identity to the amino acid sequence set forth in SEQ ID NO: 8; and
a VL having at least 80% identity to the amino acid sequence set forth in SEQ ID NO: 11.

18. The use according to any one of claims 1 to 17, wherein the anti-IL-17RB antibody comprises: a VH of the amino acid sequence set forth in SEQ ID NO: 8, and a VL of the amino acid sequence set forth in SEQ ID NO: 11.

19. The use according to any one of claims 1 to 18, wherein the anti-IL-17RB antibody further comprises: a heavy chain constant region having at least 80% identity to the amino acid sequence set forth in SEQ ID NO: 18 or 14.

20. The use according to claim 19, wherein the anti-IL-17RB antibody further comprises a heavy chain constant region set forth in SEQ ID NO: 18.

21. The use according to any one of claims 1 to 20, wherein the anti-IL-17RB antibody further comprises a human kappa chain constant region.

22. The use according to claim 21, wherein the anti-IL-17RB antibody further comprises: a CL having at least 80% identity to the amino acid sequence set forth in SEQ ID NO: 16.

23. The use according to any one of claims 1 to 22, wherein the anti-IL-17RB antibody comprises:
a heavy chain having at least 80% identity to the amino acid sequence set forth in SEQ ID NO: 19, 15, 20, or 21; and
a light chain having at least 80% identity to the amino acid sequence set forth in SEQ ID NO: 17.

24. The use according to claim 23, wherein the anti-IL-17RB antibody comprises:
a heavy chain having at least 80% identity to the amino acid sequence set forth in SEQ ID NO: 19; and
a light chain having at least 80% identity to the amino acid sequence set forth in SEQ ID NO: 17.

25. The use according to claim 24, wherein the anti-IL-17RB antibody comprises: a heavy chain set forth in SEQ ID NO: 19; and a light chain set forth in SEQ ID NO: 17.

26. The use according to any one of claims 1 to 25, wherein the anti-IL-17RB antibody is of an IgG1, IgG2, IgG3, or IgG4 isotype.

27. The use according to any one of claims 1 to 26, wherein the antigen-binding fragment is selected from a Fab fragment, a Fab' fragment, an F(ab')2 fragment, an Fd fragment, an Fv fragment, a dAb fragment, an isolated CDR region, an scFv, and a nanobody.

28. The use according to any one of claims 1 to 27, wherein the pharmaceutical composition further comprises a pharmaceutically acceptable excipient.

29. The use according to any one of claims 1 to 28, wherein the pharmaceutical composition further comprises an additional therapeutic agent comprising: an anti-allergic agent, an analgesic agent, an anti-inflammatory agent, an anesthetic agent, and an antipyretic agent.

30. The use according to any one of claims 1 to 29, wherein the pharmaceutical composition further comprises an additional therapeutic agent for atopic dermatitis comprising at least one of a glucocorticoid, a calcineurin inhibitor, an antibiotic, an antipruritic agent, an antihistamine agent, a JAK inhibitor, a PDE4 inhibitor, or calamine lotion.
